# EUROPEAN PATENT APPLICATION

(11) **EP 3 202 316 A1**
(43) Date of publication of application: **09.08.2017**
(21) Application number: 16154150.3
(22) Date of filing: 03.02.2016
(51) Int. Cl.: A61B 5/0408

(54) **SUCTION-ATTACHMENT STRUCTURE**

(71) Applicant: King's Metal Fiber Technologies Co., Ltd., 42060 Taichung City (TW)
(72) Inventor: KANG, Yu Hsun, 10451 Taipei City (TW); CHEN, Reng Sho, 10451 Taipei City (TW); HOU, Jaang Jiun, 10451 Taipei City (TW); LIAO, Shu Fen, 10451 Taipei City (TW); WANG, Hao Chen, 10451 Taipei City (TW)
(74) Representative: Cabinet Chaillot

(57) **Abstract**

A suction-attachment structure includes an electrically conducting unit and a suction-attaching member. The suction-attaching member includes a base. The base has an edge from which a wall extends. The wall has an upper end forming an attaching face. The electrically conducting unit is coupled to the base of the suction-attaching member. The attaching face formed at the upper end of the wall defines a suction-attaching configuration to allow the suction-attaching member to securely hold the electrically conducting unit in position. As such, the electrically conducting unit is prevented from displacing during an operation of detection and contact engagement with a surface layer of a human body can be securely maintained for carrying out detection of physiological signals.

## Description

### BACKGROUND OF THE INVENTION

### 1. Field of the Invention

The present invention relates generally to a suction-attachment structure, and more particularly to a combined arrangement of an electrically conducting unit and a suction-attaching member that helps prevent undesired displacement of the electrically conducting unit in carrying out a detection operation and also helps maintain a state of being in contact engagement with a surface layer of a human body so as to applicable to a physiological detection device, a tilt detection device, and the likes.

### 2. Description of Related Art

Major technical manufacturers have recently devoted themselves to the study of combining electrode plates with a woven or knitted fabric product in order to detect physiological signals when the fabric product is worn for the purposes of getting aware of physical function conditions and also for monitoring and recording purposes.

However, when the electrode plates are put in contact with the surface layer of a human body, such electrode plates are generally fixed on the fabric. Thus, with the movement and activity of the human body, the electrode plates are often caused to undergo displacement, leading to unexpected interruption of signals or generation of abnormal signals during the detection of physiological signals conducted by the electrode plates on the surface layer of the human body. This makes it necessary to re-adjust the location of the electrode plates, or alternatively, the human body must keep still in order to allow for smooth operations of detection of physiological signals by the electrode plates.

Thus, in view of the above problems, the present inventor wishes to propose a suction-attachment structure that prevents electrode plates from displacing during a detection operation and allows for easy operation by a user for assembly. This is the motivation of the invention made by the present inventor.

### SUMMARY OF THE INVENTION

The primary object of the present invention is to provide a suction-attachment structure, which comprises a combined arrangement of an electrically conducting unit and a suction-attaching member. The suction-attaching member comprises a base. The base has an edge from which a wall extends. The wall has an upper end forming an attaching face. The electrically conducting unit is coupled to the base of the suction-attaching member. The attaching face at the upper end of the base define a suction-attaching configuration to allow the suction-attaching member to securely hold the electrically conducting unit in position, so as to prevent the electrically conducting unit from displacing during an operation of detection and to securely maintain contact engagement with a surface layer of a human body for effectively carrying out detection of physiological signals thereby improving overall utilization.

Another object of the present invention is to provide a suction-attachment structure, which comprises a suction-attaching member comprising at least one extension section extending from an edge of a base. The extension section has an edge from which a wall is extended. The extended wall has an upper end forming an attaching face. The extension section comprises an electrically-conductive extension unit arranged therein. The electrically-conductive extension unit is extended from an electrically conducting unit arranged in the suction-attaching member so as to increase the contact area thereof with a surface layer of a human body to help improve accuracy of physiological signal detection thereby improving overall accuracy.

A further object of the present invention is to provide a suction-attachment structure, which comprises a suction-attaching member comprising at least one extension section extending from an edge of the base. The suction-attaching member and the extension section are formed of electrically conductive rubber to improve the attachability thereof and also to allow the electrically conducting unit arranged in the suction-attaching member to extend into the extension section in order to improve the signal magnitude of a physiological signal detected thereby for improving the detection performance, thereby improving overall utilization.

To achieve the above objects, the present invention provides a suction-attachment structure, which comprises an electrically conducting unit and a suction-attaching member. The suction-attaching member comprises a base. The base has an edge from which a wall extends. The wall has an upper end forming an attaching face. The electrically conducting unit is coupled to the base of the suction-attaching member. The attaching face formed at the upper end of the wall defines a suction-attaching configuration to allow the suction-attaching member to securely hold the electrically conducting unit in position.

The above and other objects and advantages of the present invention can be fully understood from the following detailed description, and the attached drawings, given with reference to selected embodiments.

Certainly, some of the components of the present invention, or arrangements of components may be different; however, the selected embodiment will be described, in details, in the specification and illustrated in the attached drawings.

### BRIEF DESCRIPTION OF THE DRAWINGS

FIG. 1 is a perspective view showing a primary embodiment of the present invention;
FIG. 2 is a cross-sectional view showing the primary embodiment of the present invention;
FIG. 3 is a perspective view showing the primary embodiment of the present invention having a male fastening member;
FIG. 4 is a perspective view showing the primary embodiment of the present invention having a male fastening member and a female fastening member;
FIG. 5 is a perspective view showing a second embodiment of the present invention that comprises two extension sections;
FIG. 6 is a perspective view showing the second embodiment of the present invention that comprises six extension sections; and
FIG. 7 is a perspective view showing a third embodiment of the present invention that comprises an extension section of conductive rubber.

### DETAILED DESCRIPTION OF THE PREFERRED EMBODIMENTS

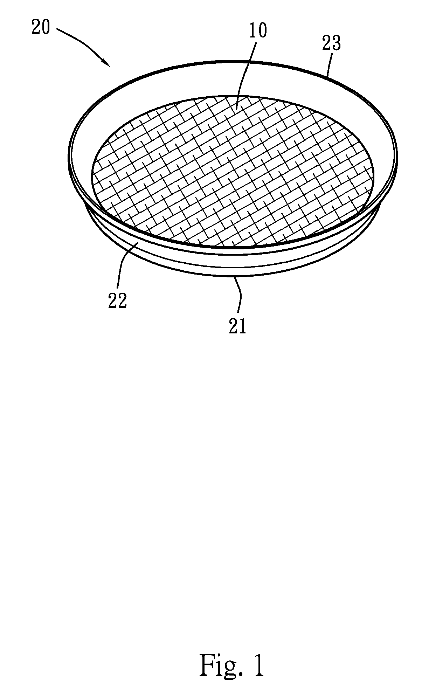

Referring to FIGS. 1-7, which are schematic views of embodiments of the present invention, the present invention provides a suction-attachment structure, of which a preferred way of embodiment is applicable to a physiological detection device, a tilt detection device, or the likes to prevent an electrically conducting unit 10 from displacing during a detection operation and to maintain a state of being in contact engagement with a surface layer of a human body in order to effectively conduct detection of physiological signals and help improve the performance of detection.

A primary embodiment of the suction-attachment structure according to the present invention generally comprises an electrically conducting unit 10 and a suction-attaching member 20 (as shown in FIG. 1), wherein the electrically conducting unit 10 can be one of a piece of electrically conductive fabric and an electrode plate (or other electrically conductive substitutes) and, with the electrically conducting unit being positionable on and set in tight engagement with human body skin, detection of a physiological signal on the surface layer of a human body can be conducted, where the physiological signal can be one of body temperature, heartbeat, pulse, and breath. The electrically conducting unit 10 can be formed by weaving or knitting a plurality of conductive fiber yarns or alternatively by weaving or knitting a plurality of non-conductive fiber yarns and a plurality of conductive fiber yarns in combination so as to provide the electrically conducting unit 10 with an effect of electrical conductivity.

Further, the suction-attaching member 20 of the present invention is made of silica gel or electrically conductive rubber. The silica gel used is particulate porous hydrated silica washed with sodium silicate added with acid and the dried, having a temperature resistance for 220-280°C and having excellent property of releasing. The silica gel is non-conductive. The conductive rubber has an effect of electrical conductivity and the conductive rubber is formed of a base material of high performance silicon rubber, added with conductive particles, such as particles of copper plated with silver, particles of aluminum plated with silver, particles of glass plated with silver, and particles of graphite plated with nickel, and assisting agents, in such a way that the conductive particles are uniformly mixed in the silicon rubber and the conductive particles are in contact engagement with each other with pressure applied thereto so as to achieve an excellent effect of electrical conductivity. In addition to the above-described silica gel and conductive rubber, the suction-attaching member 20 of the present invention may also be formed of other plastic materials involving electrical conductivity or not involving electrical conductivity, and the materials that can be used are not limited to what described or mentioned above.

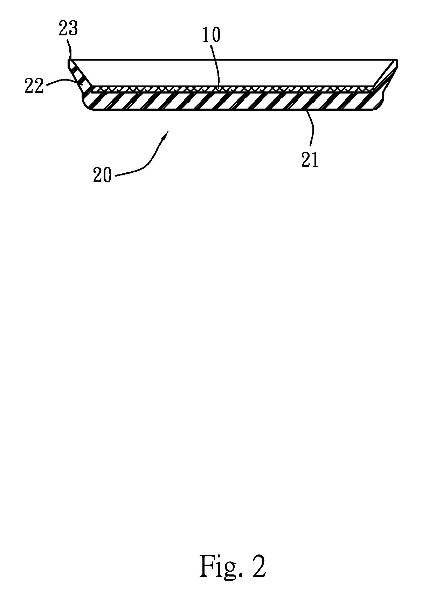

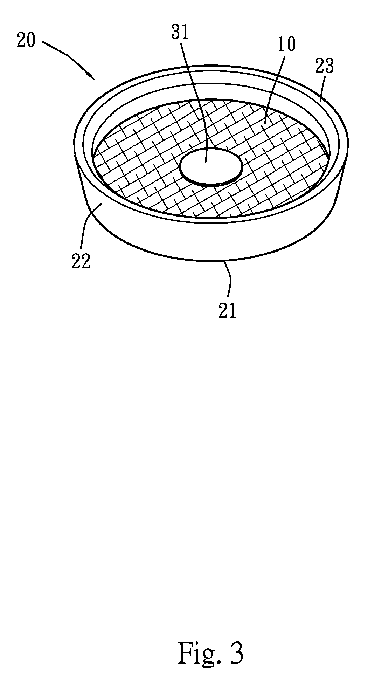

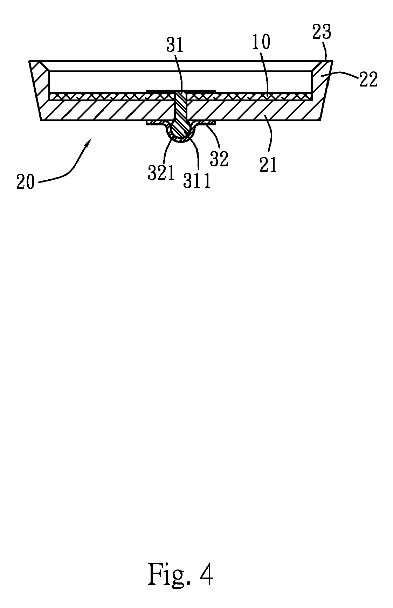

The suction-attaching member 20 comprises a base 21. The base 21 has an edge from which a wall 22 extends to form a bowl like configuration. The wall 22 has an upper end that forms an attaching face 23 (as shown in FIGS. 1 and 2). The attaching face 23 is in a flat configuration for attaching to the surface layer of a human body and a hermetically sealed area can be formed by and inside the wall 22 to prevent moisture generated by the surface layer of the human body that is located in the hermetically sealed area from being readily dissipated and to preserve the moisture in the hermetically sealed area for improving the effect of electrical conductivity. Further, the electrically conducting unit 10 is coupled to the base 21 of the suction-attaching member 20. The coupling can be achieved with various ways, such as adhesive, riveting, and nailing/screwing. A preferred form of coupling is provided below, where a male fastening member 31 and a female fastening member 32 are provided. The male fastening member 31 has a front end that is formed as a rounded projecting end 311, and the female fastening member 32 has a middle portion that is formed as a circular concave structure 321. The rounded projecting end 311 of the front end of the male fastening member 31 has a diameter hat is substantially equal to or greater than a diameter of the circular concave structure 321 formed in the middle portion of the female fastening member 32. The rounded projecting end 311 of the male fastening member 31 is penetrated through the electrically conducting unit 10 first and then put through the base 21 of the suction-attaching member 20 to finally fit into the circular concave structure 321 of the female fastening member 32, so that the male fastening member 31 is set in tight fitting engagement in the female fastening member 32 (as shown in FIGS. 3 and 4), whereby the electrically conducting unit 10 is coupled to the base 21 of the suction-attaching member 20. The attaching face 23 formed on the upper end of the wall 22 provides a suction-attaching configuration that allows the suction-attaching member 20 to maintain and hold the position of the electrically conducting unit 10 and prevent the electrically conducting unit 10 from displacing during an operation of detection to affect the detection of a physiological signal.

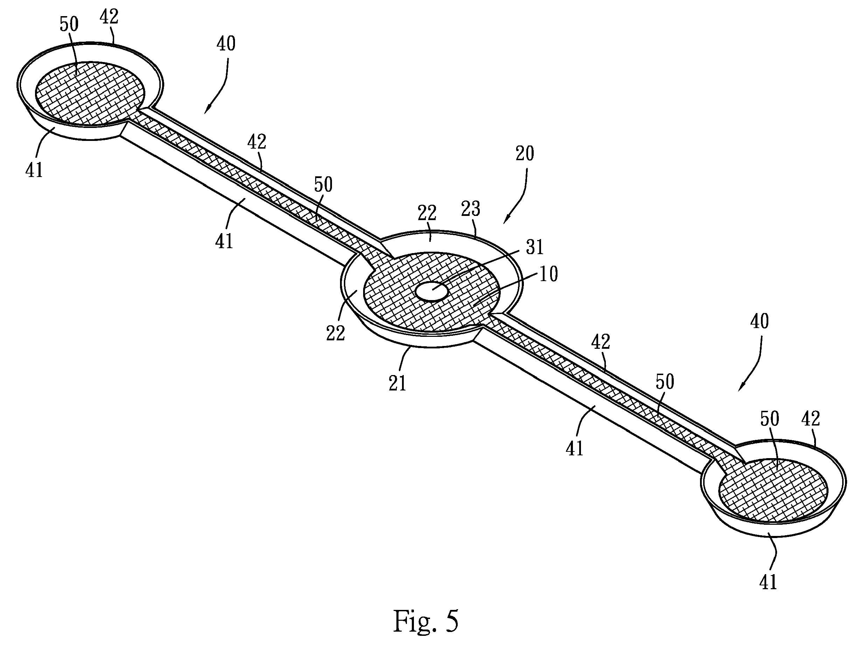

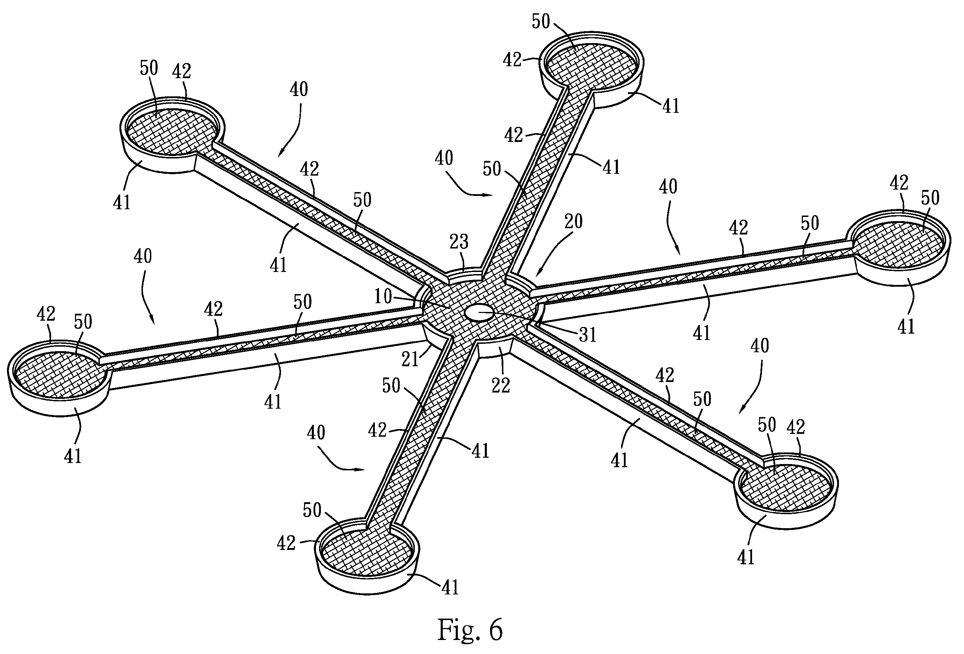

A second embodiment of the suction-attachment structure according to the present invention is formed on the basis of the electrically conducting unit 10 and the suction-attaching member 20 of the primary embodiment described above, where the electrically conducting unit 10 can be one of a piece of electrically conductive fabric and an electrode plate (or other electrically conductive substitutes) and the base 21 of the suction-attaching member 20 is structured to have at least one extension section 40 extending from an edge thereof, wherein the number of the extension section 40 is plural and can be one, two, three, ..., or six (two and six extension sections being taken as examples of the present invention) (as shown in FIGS. 5 and 6), and walls 41 extend from edges of the extension section 40 and the walls 41 so extending have an upper end forming an attaching face 42. The attaching face 42 is in a flat configuration for attaching to the surface layer of a human body. The walls 41 of the extension section 40 and the wall 22 of the suction-attaching member 20 are connected to each other for forming and delimiting a hermetically sealed area prevent moisture generated by the surface layer of the human body that is located in the hermetically sealed area from being readily dissipated and to preserve the moisture in the hermetically sealed area for improving the effect of electrical conductivity. The wall 22 of the suction-attaching member 20 and the walls 41 of the extension section 40 can be arranged in various forms, such as being vertical (as shown in FIG. 6) or inclined (as shown in FIG. 5), whereby the present invention makes use of the extension section 40 extending from the base 21 of the suction-attaching member 20 to expand the contact area thereof with the surface layer of a human body and uses the extension section 40 to help stably hold the suction-attaching member 20 in stable contact engagement with the surface layer of the human body. Further, the extension section 40 is provided therein with an electrically-conductive extension unit 50, and the electrically-conductive extension unit 50 is extended from the electrically conducting unit 10 that is arranged in the suction-attaching member 20 so as to expand the effect of the electrically conducting unit 10 and to enhance the detection capability thereof for physiological signals.

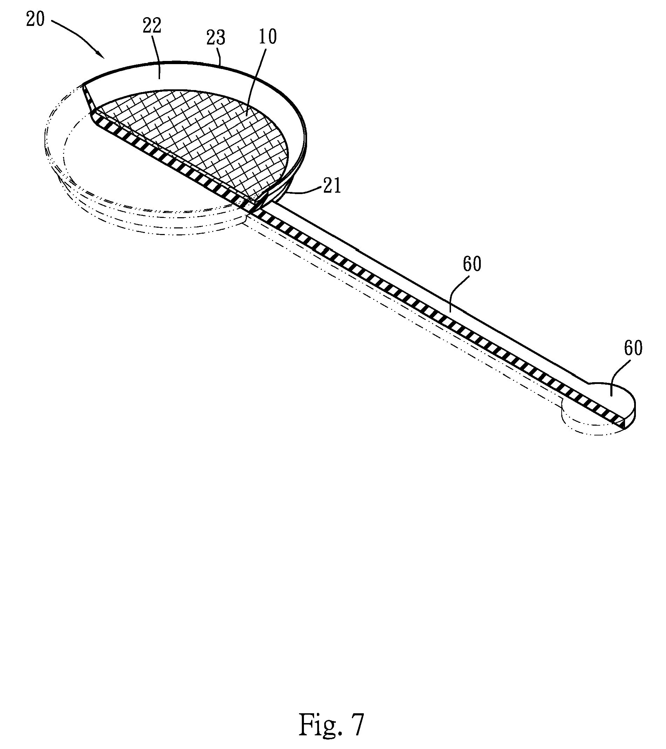

A third embodiment of the suction-attachment structure according to the present invention is formed on the basis of the electrically conducting unit 10 and the suction-attaching member 20 of the primary embodiment described above, where the electrically conducting unit 10 can be one of a piece of electrically conductive fabric and an electrode plate (or other electrically conductive substitutes) and the base 21 of the suction-attaching member 20 is structured to have at least one extension section 60 extending from an edge thereof (as shown in FIG. 7). The electrically conducting unit 10 is coupled to the base 21 of the suction-attaching member 20. The coupling can be achieved with various ways, such as adhesive, riveting, and nailing/screwing, which can be an arrangement (not shown) of a male fastening member 31 and a female fastening member 32 similar to those of the primary embodiment. In the instant embodiment, adhesive is taken as an example for achieving the coupling. Further, in the instant embodiment, the suction-attaching member 20 and the extension section 60 are formed of conductive rubber and the suction-attaching member 20 and the extension section 60 integrally formed together as a unit. With a surface of the extension section 60 being attachable to the surface layer of a human body, the electrically conducting unit 10 that is coupled to the base 21 of the suction-attaching member 20 may expand the capability of detecting a physiological signal through the suction-attaching member 20 and the extension section 60 that are made of conductive rubber and to increase the contact area thereof with the surface layer of the human body by means of the extension section 60 so that the accuracy of the physiological detection signal can be improved.

Further, in the above-described first, second, and third embodiments of the suction-attachment structure according to the present invention, the electrically conducting unit 10 can be connected to a signal transmission point (not shown) and the signal transmission point is connected via a conductor line (not shown) to a signal transmitter. The signal transmitter may comprise a wireless transmission module to transmit, in a wireless manner, the physiological signal detected and variation thereof to an electronic device (such as a smart phone, a smart tablet computer, a notebook computer, a desktop computer, and medical equipment) or cloud for easy aware of the result of detection to take measure for preventive medical treatments.

Based on the above detailed description, those skilled in the art may appreciate that the present invention can achieve the above-discussed objectives and is in complete compliance with the Patent Laws and thus, an application is filed herewith for seeking protection offered by a patent granted therefor.

However, it is noted that the above description is made only to preferred embodiments of the present invention and is not intending to limit the true scope where the present invention may be put into practice. Thus, simple and equivalent variations and modifications made on the disclosure of the specification and the attached claims are all considered within the scope of the present invention.

## Claims

1. A suction-attachment structure, comprising:
an electrically conducting unit; and
a suction-attaching member, which comprises a base, the base having an edge from which a wall extends, the wall having an upper end forming an attaching face, the electrically conducting unit being coupled to the base of the suction-attaching member, wherein the attaching face formed at the upper end of the wall defines a suction-attaching configuration for allowing the suction-attaching member to maintain and hold the electrically conducting unit in position.

2. The suction-attachment structure as claimed in Claim 1, wherein the suction-attaching member further comprises at least one extension section extending from the edge of the base, the extension section having an edge from which a wall is extended, the extended wall having an upper end forming an attaching face, the extension section comprising an electrically-conductive extension unit arranged therein, the electrically-conductive extension unit being extended from the electrically conducting unit that is arranged in the suction-attaching member.

3. The suction-attachment structure as claimed in Claim 1 or 2, wherein the suction-attaching member is formed of silica gel.

4. The suction-attachment structure as claimed in Claim 1 or 2, wherein the wall is vertical.

5. The suction-attachment structure as claimed in Claim 1 or 2, wherein the wall is inclined.

6. The suction-attachment structure as claimed in Claim 1 or 2, wherein the suction-attaching member is formed of electrically conductive rubber.

7. The suction-attachment structure as claimed in Claim 1, wherein the suction-attaching member further comprises at least one extension section extending from the edge of the base, the suction-attaching member and the extension section being formed of electrically conductive rubber.

8. The suction-attachment structure as claimed in Claim 1, wherein the electrically conducting unit is coupled to the suction-attaching member by a male fastening member and a female fastening member, the male fastening member having a front end forming a rounded projecting end, the female fastening member having a middle portion forming a circular concave structure, the rounded projecting end of the front end of the male fastening member having a diameter that is substantially equal to or greater than a diameter of the circular concave structure of the middle portion of the female fastening member, the rounded projecting end of the male fastening member penetrating through the electrically conducting unit and the base of the suction-attaching member to get fit into the circular concave structure of the female fastening member so as to tightly fit the male fastening member in the female fastening member.

9. The suction-attachment structure as claimed in Claim 1, wherein the electrically conducting unit is connected to a signal transmission point, the signal transmission point being connected via a conductor line to a signal transmitter, the signal transmitter comprising a wireless transmission module arranged therein for transmission of a signal applied thereto.

10. The suction-attachment structure as claimed in Claim 1, 2, 8, or 9, wherein the electrically conducting unit comprises one of electrically conductive fabric and an electrode plate and the electrically conducting unit is positionable against human body skin to detect a physiological signal of a surface layer of a human body, the physiological signal being one of body temperature, heartbeat, pulse, and breath.
